## Europäisches Patentamt

## European Patent Office

## Office européen des brevets

(19)

(11) Veröffentlichungsnummer: **0 170 787**
**B1**

(12) **EUROPÄISCHE PATENTSCHRIFT**

(45) Veröffentlichungstag der Patentschrift:
**20.07.88**

(51) Int. Cl.⁴: **A 61 B 17/58**

(21) Anmeldenummer: **85105224.1**

(22) Anmeldetag: **29.04.85**

(54) Vorrichtung zum Fixieren von Röhrenknochenfrakturen mit Knochenmarknagel und Verriegelungsbolzen.

(30) Priorität: **14.05.84 CH 2385/84**

(43) Veröffentlichungstag der Anmeldung:
**12.02.86 Patentblatt 86/7**

(45) Bekanntmachung des Hinweises auf die Patenterteilung:
**20.07.88 Patentblatt 88/29**

(84) Benannte Vertragsstaaten:
**BE DE FR IT SE**

(56) Entgegenhaltungen:
**EP-A-0 029 752**
**CH-A-572 737**
**DE-B-1 248 228**
**US-A-3 709 218**

(73) Patentinhaber: **Synthes AG Chur, Grabenstrasse 15,
CH- 7002 Chur (CH)**

(72) Erfinder: **Klaue, Kaj, Dr. med., Spiegelstrasse 64,
CH- 3028 Spiegel bei Bern (CH)**

(74) Vertreter: **Klingseisen, Franz, Dipl.- Ing., Dr. F.
Zumstein sen. Dr. E. Assmann Dr. F. Zumstein
jun. Dipl.- Ing. F. Klingseisen Bräuhausstrasse 4,
D-8000 München 2 (DE)**

EP 0 170 787 B1

## Beschreibung

Gegenstand der Erfindung ist eine Vorrichtung zum Fixieren von Röhrenknochenfrakturen mit Knochenmarknagel und Verriegelungsbolzen.

Bei der bisher üblichen Marknagelverriegelung wird nach dem Einschlagen des Marknagels im Bereich der Nageleinschlagstelle ein erster, meist schräg geführter Querbolzen mit selbstschneidendem Endgewinde durch den Knochen und eine vorgebohrte Ausnehmung im Nagel hindurch eingebracht, wonach ein oder zwei weitere Querbolzen in im andern Endbereich des Nagels vorgesehene Öffnungen eingebracht werden. Solcherart verriegelte Marknägel führen meist zu einer einwandfreien Fixierung der Knochenfraktur, wobei vorausgesetzt ist, daß die im Nagel vorgesehenen Aufnahmeöffnungen für die Querbolzen dem Querschnitt dieser Bolzen genau angepaßt sein müßen, was anderseits ein genau lagerichtiges Einführen der Querbolzen in die verdeckten Aufnahmeöffnungen des eingeschlagenen Nagels voraussetzt. Es ist bekannt, daß dies zu erheblichen Schwierigkeiten führt, weshalb mit relativ komplizierten Zielgeräten unter dauernder Mitverwendung röntgenoptischer Mittel mit Bildverstärkung gearbeitet werden muß; letzteres ist nicht nur umständlich, sondern kann zu unerwünscht starker Strahlenbelastung des Patienten führen.

Zur Vermeidung dieser Nachteile bezweckt die vorliegende Erfindung die Schaffung einer Vorrichtung der genannten Art, die eine einwandfreie Marknagelverriegelung ergibt und dabei ein genaues relativ schnelles Arbeiten des Chirurgen unter größtmöglicher Schonung des betroffenen Patienten ermöglicht.

Zu diesem Zweck ist die erfindungsgemäße Vorrichtung dadurch gekennzeichnet, daß ein erster der Erstverriegelung dienender Querbolzen mit einer Aufnahmepartie versehen ist, mit welcher die mit einem Einführteil versehene Spitzenpartie des Marknagels durch Längsverschieben des letzteren senkrecht zum ruhenden Querbolzen in Eingriff bringbar ist.

Dank dieser Ausbildung ist es möglich, den Marknagel erst nach dem korrekten Setzen eines Erstverriegelungs- oder Zielbolzens in die Knochenbohrung einzuführen, bis die entsprechend ausgebildete Spitzenpartie des Nagels auf die Aufnahmepartie des Bolzens trifft. Da dem Nagel die Eindringrichtung durch die Bohrung im Knochen weitgehend vorgegeben ist und das korrekte, etwa diametral zu dieser Bohrung erfolgende Setzen des Bolzens keinerlei Schwierigkeiten bereitet, findet der Einführteil der Nagelspitzenpartie auch bei geringfügiger Abweichung des Nagels von seiner Ideallage die Aufnahmepartie des Querbolzens praktisch automatisch.

Im Folgenden ist die Erfindung anhand der Zeichnung beispielsweise erläutert.

In der Zeichnung zeigen:

Fig. 1 in Wirkungslage ein Beispiel der aus Marknagel und drei Verriegelungsbolzen bestehenden Vorrichtung zur Fixierung einer Oberschenkelfraktur,

Fig. 2 eine bezüglich Fig. 1 um 90° gedrehte Ansicht,

Fig. 3 bis 6 je eine Ansicht analog Fig. 1 bzw. 2, verschiedene Arbeitsschritte während der Verriegelungsnagelung zeigend,

Fig. 7 bis 11 schaubildlich die Spitzenpartie je einer Variante des Marknagels der Vorrichtung,

Fig. 12 eine Stirnansicht zu Fig. 11,

Fig. 13 bis 19 schaubildlich je eine Variante des Marknagels mit Erstverriegelung,

Fig. 20 schaubildlich eine weitere Variante des Beispiels analog Fig. 2 mit Zielbolzen und Bohrbüchsenträger,

Fig. 21 in Seitenansicht die Spitzenpartie des gegenüber Fig. 20 um 90° gedrehten Marknagels, und

Fig. 22 bis 29 in Seitenansicht je eine Variante der Spitzenpartie des Marknagels mit Verriegelungsbolzen.

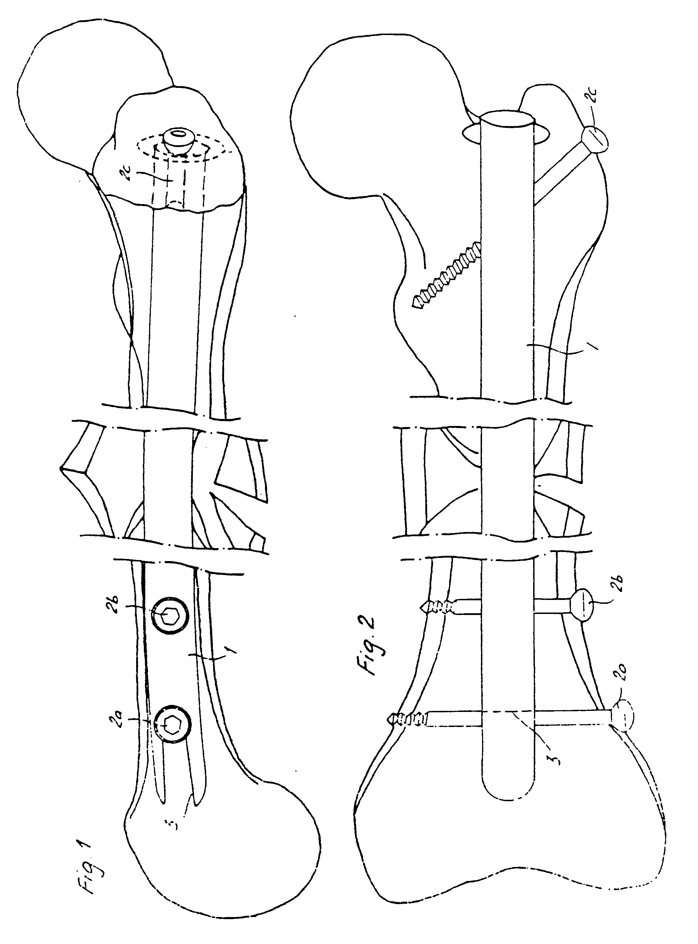

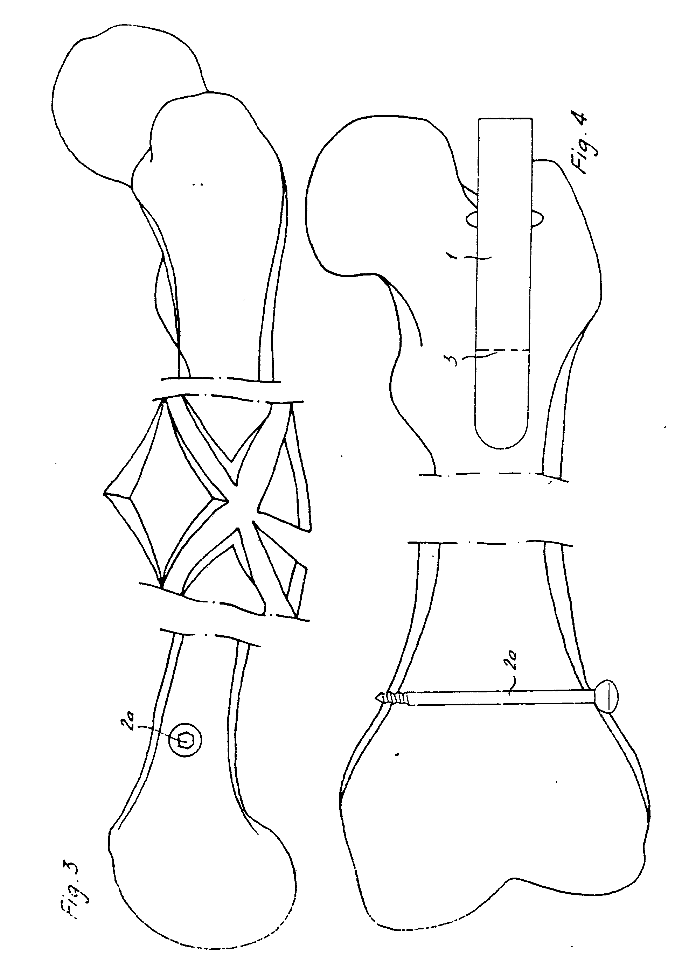

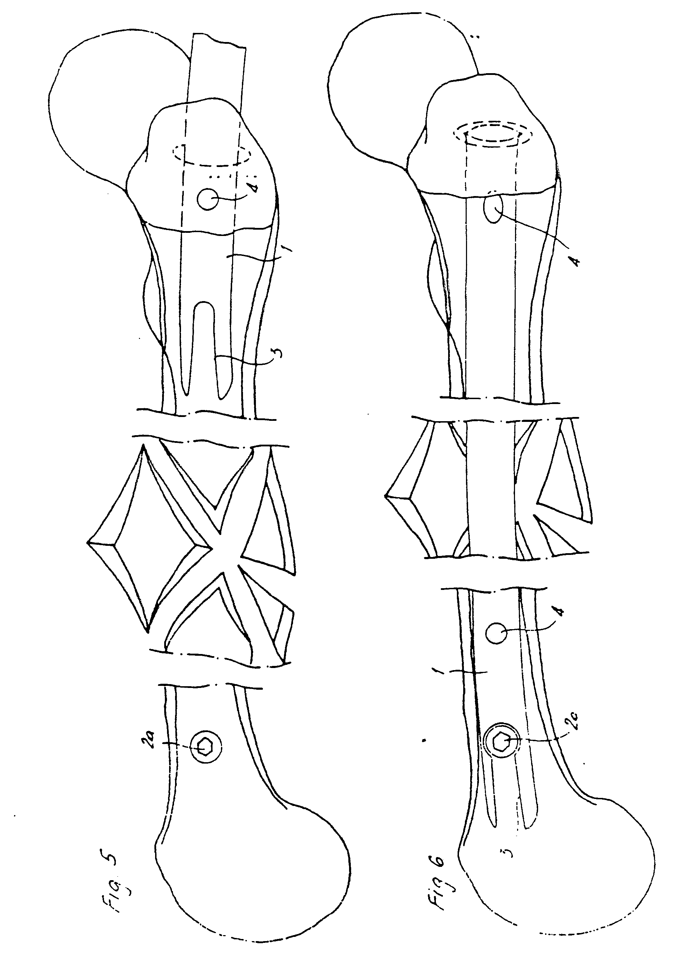

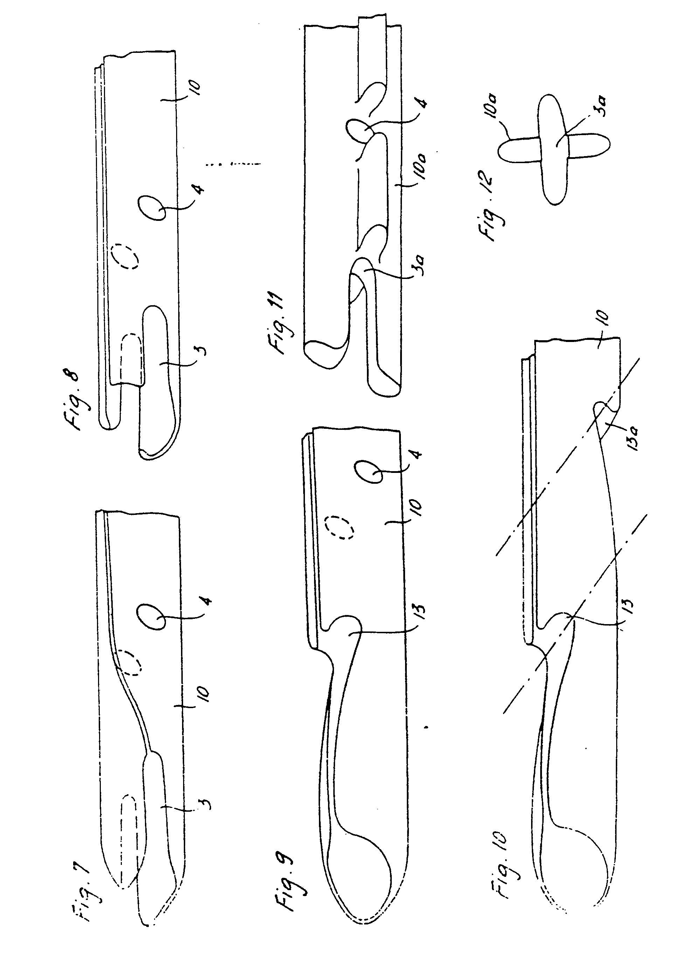

Die in den Fig. 1 und 2 dargestellte Vorrichtung besteht aus einem Marknagel 1 und mehreren, hier drei, Verriegelungsbolzen 2a, 2b, 2c mit z. B. selbstschneidendem Endgewinde. Zur Aufnahme des Erstverriegelungsbolzens 2a besitzt die Spitzenpartie des Marknagels 1 einen dem Schaftdurchmesser des als Aufnahmepartie dienenden Schaftes dieses Bolzens 2a entsprechenden Einführschlitz 3, dessen Mündung zweckmäßig etwas erweitert ist. Für die beiden andern Verriegelungsbolzen 2b, 2c ist der Marknagel 1 in üblicher Weise mit einer diametralen bzw. einer Schrägbohrung versehen. Die Anwendung dieser Vorrichtung ergibt sich aus den Figuren 3 bis 6. Nach den üblichen Vorarbeiten (Reposition, Aufbohren etc) wird zuerst an der vorbestimmten Stelle (distal) der der Erstverriegelung dienende Verriegelungsbolzen 2a in den Knochen eingesetzt (Fig. 3). Anschließend wird der Marknagel 1 von der proximalen Einführöffnung her in die Markbohrung eingeführt (Fig. 4 und 5); der eine relativ weite Öffnung aufweisende, fischmaulartige Einführschlitz 3 der Spitzenpartie des Nagels 1 nimmt dabei den als Aufnahmepartie dienenden, die Markbohrung durchsetzenden Schaft des Erstverriegelungsbolzens 2a auf, bis dieser am entsprechend gerundeten Schlitzgrund anliegt (Fig. 6). Geringfügige, oft unvermeidbare Abweichungen des Nagelverlaufs bezüglich der Lage des Bolzens 2a werden beim Einlaufen des Bolzens 2a in den sich auf den Bolzendurchmesser nach innen verengenden Einführschlitz 3 zwangsläufig ausgeglichen. Dank dieser Erstverriegelung des Marknagels 1 sind Axial- und Umfangslage der für das zum Erstverriegelungsbolzen achsparallele Einsetzen der zusätzlichen Verriegelungsbolzen 2b und 2c vorzusehenden Stellen bzw. Nagellöcher 4 von außen exakt bestimmbar. Damit ist eine Überwachung der einzelnen Arbeitsgänge praktisch nur während der letzten Phase der Nageleinführung erforderlich, und ein den Patienten unnötig belastendes Suchen der Nagelöffnung für die Erstverriegelung mit einem Querbolzen ist unnötig.

Im Vorangehenden wurde vorausgesetzt, daß der vor der Marknageleinführung zu setzende Querbolzen 2a gleichzeitig der Erstverriegelungsbolzen ist. An dessen Stelle kann aber auch ein sogenannter Zielbolzen verwendet werden, der nach dem korrekten Eingriff der geschlitzten Spitzenpartie des Marknagels entfernt und nun problemlos durch den Erstverriegelungsbolzen ersetzt werden kann.

Das anhand der Fig. 1 bis 6 beschriebene Prinzip der erfindungsgemäßen Vorrichtung bzw. deren Verwendung kann bezüglich Ausbildung der Nagelspitzenpartie bzw. der Aufnahmepartie des vorausgehend zu setzenden Querbolzens verschiedentlich abgewandelt werden. Voraussetzung aber ist stets, daß der Marknagel nur durch seine Längsverschiebung rechtwinklig zum Querbolzen mit diesem in Eingriff kommen kann, was zwangsläufig zu der vorbeschriebenen, bezüglich Zeit- und Überwachungsaufwand bzw. Patientenbelastung vorteilhaften Verwendungsart führt.

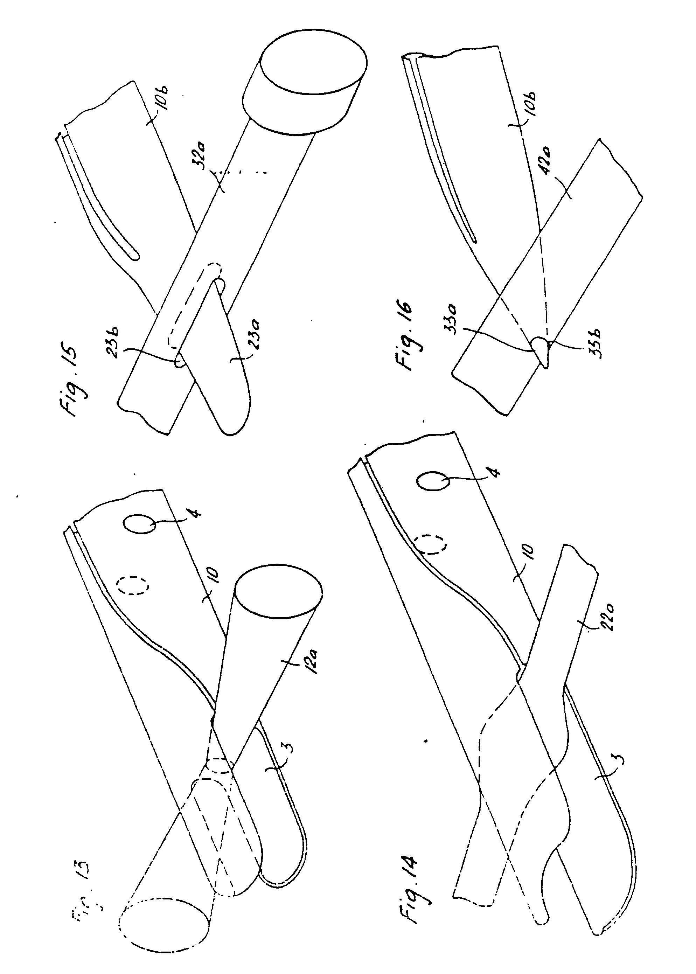

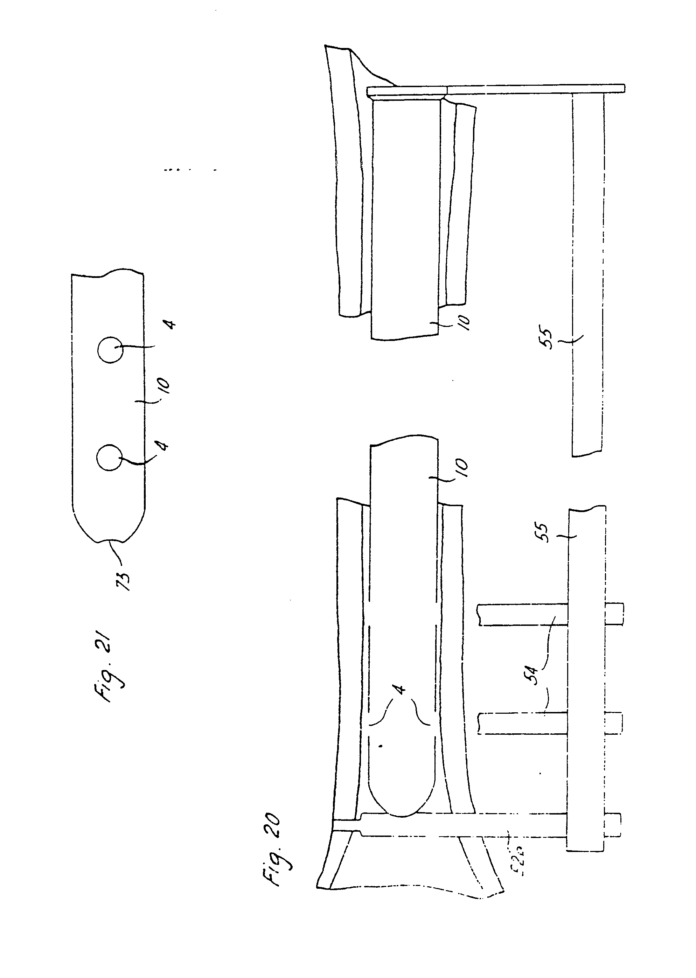

Verschiedene Ausführungsvarianten des Marknagels und der Verriegelungsbolzen sind in den Fig. 7 bis 29 dargestellt. So zeigen die Fig. 7 bis 10 und 13, 14 einen in an sich bekannter Weise als zylindrisches, durchgehend längsgeschlitztes Rohr ausgebildeten Marknagel 10. Während bei den Varianten nach Fig. 7, 8, 13 und 14 die Spitzenpartie des Nagels einen etwa achsialgeführten, mindestens einlaßseitig relativ weiten Einführschlitz 3 aufweist, ist bei den Varianten nach Fig. 9 und 10 die Spitzenpartie des Nagels auf der den Längsschlitz aufweisenden Seite etwas abgeflacht und mit einer frontseitig offenen hinterschnittenen Einführnut 13 versehen; während für das Einsetzen eines weiteren Verriegelungsbolzens bei der Variante nach Fig. 9 analog den Ausführungen nach Fig. 1 bis 8 eine Querbohrung vorgesehen ist, besitzt der Marknagel 10 gemäß Fig. 10 auf der der Einführnut 13 gegenüberliegenden Seite eine zweite, axial entsprechend zurückgesetzte Einführnut 13a. Bei Verwendung der in den Fig. 9 und 10 gezeigten Varianten wird nach dem Setzen des Erstverriegelungsbolzens die Spitzenpartie des Marknadels diesen Bolzen unterfahren, bis dieser vollständig in der Einführnut 13 gefangen ist. Die Fig. 11 und 12 zeigen, daß auch ein im Querschnitt anders, z. B. als Hohlkreuz, ausgebildeter Marknagel 10a mit einem Einführschlitz 3 versehen sein kann.

Die Fig. 13 und 14 zeigen, daß zur Erstverriegelung anstelle eines zylindrischen Querbolzens auch ein doppelkonischer Bolzen 12a mit mittig angeordnetem kleinstem Querschnitt, oder ein mittig verdickter Querbolzen 22a verwendet werden kann. Beide Varianten bedingen zwar eine dem größten Bolzenquerschnitt entsprechende, relativ große Bohrung für das Einsetzen des Bolzens, sichern aber anderseits den Nagel gegen seitliches Verlaufen und bewirken somit beim Auftreffen der Nagelspitzenpartie eine Art Zentrierung des Nagels.

Bei allen vorangehend beschriebenen Ausführungen ist vorausgesetzt, daß der erstzusetzende Verriegelungsbolzen mit einer als Aufnahmeteil dienenden Schaftpartie in einen Einführschlitz bzw. eine Einführnut der Spitzenpartie des Marknagels eingeführt wird. Die Fig. 15 und 16 zeigen jedoch, daß auch eine Umkehrung dieser Eingriffsart möglich ist. So besitzt bei der in Fig. 15 gezeigten Variante der Schaft des Erstverriegelungsbolzens 32a einen als Aufnahmeteil dienenden Längsschlitz 23b, in welchen ein entsprechend flachgedrückter, nach vorn zu einer schmalen Zunge auslaufender Einführteil 23a des z. B. als längsgeschlitztes Rohr ausgebildeten Marknagels 10b eingeführt wird. Analog ist bei der Variante nach Fig. 16 im Schaft des Erstverriegelungsbolzens 42a ein konisches Querloch 33b vorgesehen, in welches beim Einführen des Marknagels 10b dessen als zu einem spitz auslaufenden Konus ausgebildeter Einführteil 33a eindringt. Zur Erhöhung der Bolzenfestigkeit kann die durch den Schlitz 23b bzw. das Konusloch 33b geschwächte Schaftpartie des Querbolzens auch etwas verdickt sein.

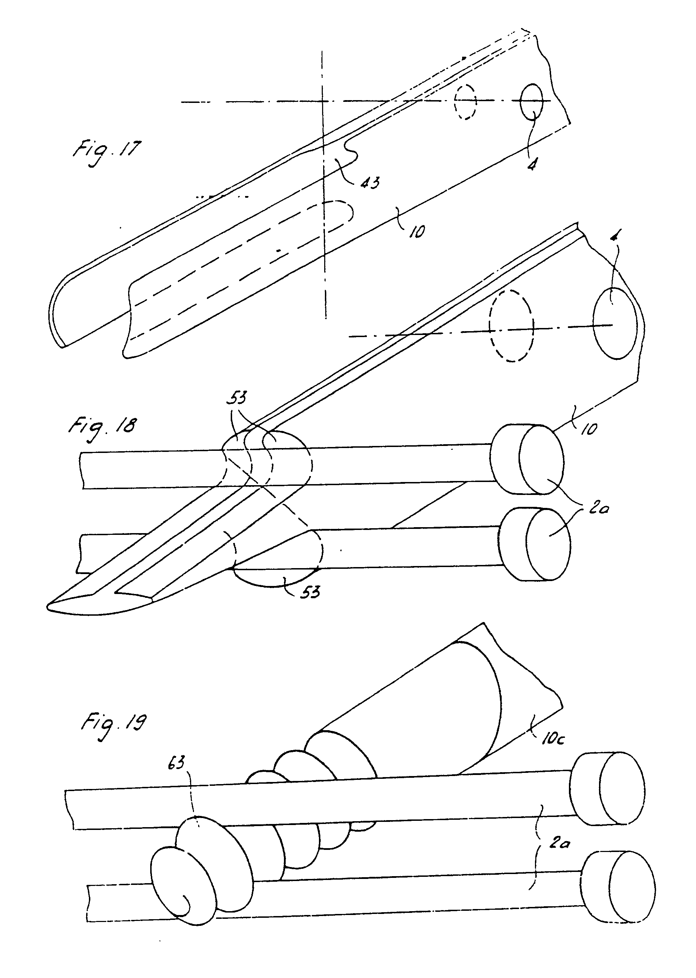

Die vorangehend beschriebenen und gezeichneten Varianten setzen alle voraus, daß die Zweit- bzw. Erstverriegelung durch einen oder mehrere zusätzliche Querbolzen achsparallel zum Erstverriegelungsbolzen erfolgt. Dies ist aber keineswegs zwingend. Wie Fig. 17 zeigt, ist es auch möglich, die Nagelbohrungen 4 für die zusätzlichen Querbolzen rechtwinklig zur Ebene des als Einführteil dienenden Frontschlitzes 43 vorzusehen. Auch in diesem Fall aber ist das exakte Bestimmen der Einsatzstellen bzw. Nagellöcher 4 für die weiteren Verriegelungsbolzen nach dem bis zum Anschlagen des Schlitzgrundes der Nagelspitzenpartie am Erstverriegelungsbolzen mittels eines geeigneten Meßgerätes ohne weiteres möglich.

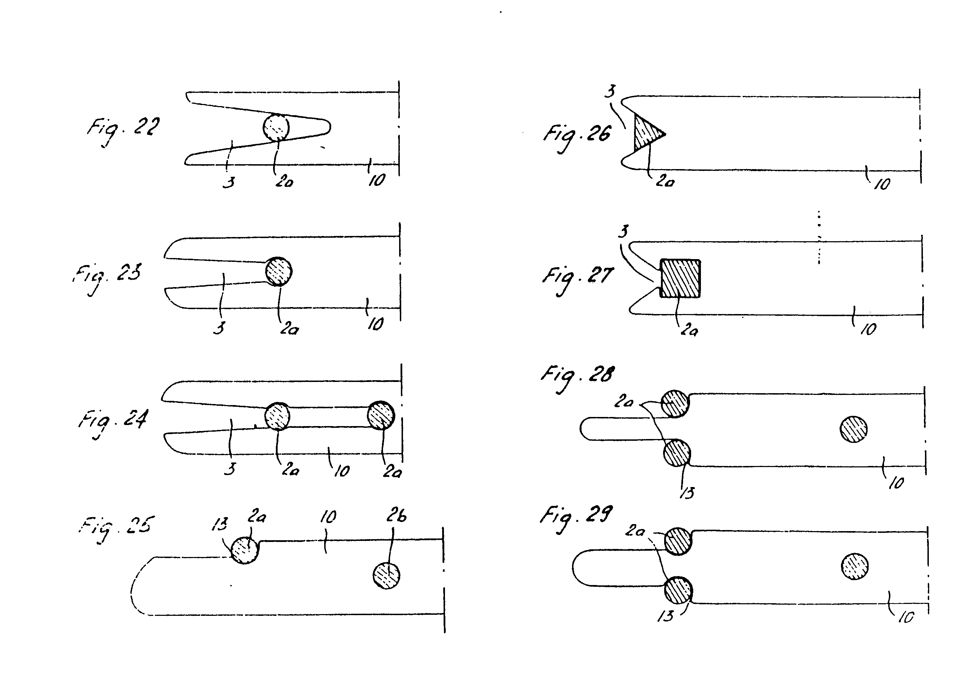

Im Vorangehenden wurde angenommen, daß zur Erstverriegelung nur ein einziger Querbolzen vorzusehen sei. Wie aber die Varianten nach den Fig. 8 und 9 zeigen, lassen sich zu diesem Zweck auch zwei Erstverriegelungsbolzen 2a in geringem Abstand voneinander achsparallel in einer zur Markbohrung rechtwinkligen Ebene in den Knochen einsetzen. Gemäß Fig. 18 ist die Spitzenpartie 53 des Marknagels 10 in diesem Fall keilförmig abgeflacht und geht auf beiden Flachseiten mit je einer dem Querschnitt der Verriegelungsbolzen 2a entsprechend gerundeten Schulter 53 in die zylindrische Nabenpartie über. Der Abstand der vor Einführung des Nagels 10 gesetzten Bolzen 2a ist so gewählt, daß bei vollständig eingeführtem Nagel die Schultern 53 an den beiden Bolzen 2a anliegen. Demgegenüber ist die Spitzenpartie des in Fig. 19 gezeigten zylindrischen Marknagels 10c mit einem als Einführteil dienenden Gewindeteil 63 versehen, dessen Kerndurchmesser dem Abstand der Bolzen 2a entspricht und fest zwischen die Erstverriegelungsbolzen 2a eindrehbar ist. Es ist dabei denkbar, daß nach dem Setzen der beiden Bolzen 2a zuerst der separate Gewindeteil 63 zwischen die Bolzen eingedreht und erst nachträglich der mit diesem Gewindeteil z. B. verschraubbare Nagelschaft eingeführt wird.

Eine weitere Ausführungsvariante ist in den Fig. 20, 21 dargestellt. Die leicht verjüngte Spitzenpartie des hier vorgesehenen Marknagels 10 ist mit einer als Einführteil dienenden Querrinne 73 versehen, die zum Zusammenwirken mit einem vor dem Einführen des Nagels in die Markbohrung gesetzten Zielbolzen 52a bestimmt ist. Dieser zweckmäßig gewindelose Zielbolzen könnte aber auch ein normaler Erstverriegelungsbolzen sein. Unter ständigem Messen der Eindringtiefe, was mittels eines an sich bekannten Tiefenmeßgeräts möglich, ist, wird der Nagel soweit eingeschlagen, bis er mit seiner Einführrinne 73 auf den Zielbolzen 52a trifft; durch leichtes Drehen des Nagels läßt sich der sichere Eingriff des Zielbolzens in die Rinne ohne weiteres gewährleisten. Auf einem am Einschlagende des Nagels befestigten Teleskoprohr 55 lassen sich nun die zum Bohren der Knochenöffnungen für das Einsetzen der Verriegelungsbolzen in die entsprechenden Nagellöcher 4 erforderlichen Bohrhülsen 54 ohne weiteres exakt positionieren. Nach dem Einführen der Verriegelungsbolzen kann der Zielbolzen 52a entfernt und z. B. durch einen normalen Verriegelungsbolzen ersetzt werden.

Aus dem Vorangehenden ist ersichtlich, daß sowohl bezüglich Ausbildung und Anordnung des Einführteils des Marknagels als auch bezüglich Aufnahmeteil, Art, Zahl und Lage des vorgängig der Einführung des Nagels zu setzenden Querbolzens verschiedene Varianten möglich sind. In den Fig. 22 bis 29 sind nochmals einige dieser Varianten schematisch dargestellt. Ihnen allen ist gemeinsam, daß eine der Erstverriegelung dienende formschlüßige Verbindung von Nageleinführteil und Querbolzen-Aufnahmeteil problemlos möglich ist, da der in der Knochenmarkbohrung sich beim Einführen achsial bewegende Marknagel mit seinem Einführteil zwangsläufig auf den verdeckt in der Markbohrung liegenden Aufnahmeteil des vorangehend gesetzten Querbolzens trifft. Nach dieser erfolgten Erstverriegelung ist die genaue Lage der den weiteren Nagellöchern zur Einführung weiterer Verriegelungsbolzen entsprechenden Bohrstellen von außen ohne weiteres bestimmbar. Die einzelnen Elemente der Vorrichtung, d. h. Marknagel und Verriegelungsbolzen, sind nicht komplizierter als jene der bekannten Vorrichtungen, ermöglichen aber ein weit schnelleres und insbesondere auch für den Patienten erheblich weniger belastendes Arbeiten.

## Patentansprüche

1. Vorrichtung zum Fixieren von Röhrenknochenfrakturen mit Knochenmarknagel und Verriegelungsbolzen, dadurch gekennzeichnet, daß ein erster der Erstverriegelung dienender Querbolzen mit einer Aufnahmepartie versehen ist, mit welcher die mit einem Einführteil versehene Spitzenpartie des Marknagels durch Längsverschieben des letzteren senkrecht zum ruhenden Querbolzen in Eingriff bringbar ist.

2. Vorrichtung nach Anspruch 1, dadurch gekennzeichnet, daß die Spitzenpartie des Marknagels (1; 10) einen vom Einführende des Nagels her offenen Längsschlitz (3) oder eine Querrinne (73) aufweist, in welchen Längsschlitz bzw. in welche Querrinne der als Aufnahmepartie dienende Schaftteil des Querbolzens (2a) paßt.

3. Vorrichtung nach Anspruch 1, dadurch gekennzeichnet, daß die Spitzenpartie des Marknagels (10) wenigstens eine vom Einführende des Nagels her offene Quernut (13; 13a) aufweist, in welche der als Aufnahmepartie dienende Schaftteil des Querbolzens (2a) paßt.

4. Vorrichtung nach Anspruch 2 oder 3, dadurch gekennzeichnet, daß der Marknagel durchgehende Querlöcher (4) für weitere Querbolzen (2b, 2c) aufweist, wobei die Axen dieser Querlöcher in einer Achsialebene des Marknagels liegen, wobei die Ebene des Längsschlitzes (3; 43) bzw. die Axe der Quernut (13; 13a) der Spitzenpartie des Marknagels (1; 10) in oder parallel oder rechtwinklig zu dieser Achsialebene verläuft.

5. Vorrichtung nach einem der Ansprüche 1 bis 4, dadurch gekennzeichnet, daß der Aufnahmeteil des Schaftes des Querbolzens (2a) gegenüber den übrigen Querbolzen-Schaftteilen im Querschnitt verdickt oder verdünnt ist.

6. Vorrichtung nach einem der Ansprüche 1 bis 4, dadurch gekennzeichnet, daß der die Aufnahmepartie aufweisende Querbolzen (2a) ein mit Endgewinde versehener Erstverriegelungsbolzen ist.

7. Vorrichtung nach einem der Ansprüche 1 bis 4, dadurch gekennzeichnet, daß der die Aufnahmepartie aufweisende Querbolzen ein durch einen Erstverriegelungsbolzen (2a) ersetzbarer Zielbolzen (52a) ist.

8. Vorrichtung nach Anspruch 1, dadurch gekennzeichnet, daß der Schaft des der Erstverriegelung dienenden Querbolzens (32a; 42a) als Aufnahmepartie eine durchgehende Queröffnung (23b; 33b) aufweist, in welche die als Einführteil dienende gegenüber dem Schaftquerschnitt des Marknagels abgeflachte oder konisch verjüngte Spitzenpartie (23a; 33a) des Marknagels (10b) paßt.

9. Vorrichtung nach einem der Ansprüche 1 bis 8, dadurch gekennzeichnet, daß die Aufnahmepartie des Schaftes des Querbolzens Rund- oder Mehrkantquerschnitt aufweist.

## Claims

1. Device for fixing pipe bone fractures with bone marrow nail and locking bolt, characterized in that a first transverse bolt serving for the initial locking is provided with a reception part with which the tip part of the marrow nail, which is provided with an introduction part, can be brought into engagement by longitudinal sliding of the marrow nail at right angles to the stationary transverse bolt.

2. Device according to claim 1, characterized in that the tip part of the marrow nail (1; 10) exhibits a longitudinal slit (3) open from the introduction end of the nail or a transverse channel (73), into which longitudinal slit or transverse channel the shank part serving as reception part of the transverse bolt (2a) fits.

3. Device according to claim 1, characterized in that the tip part of the marrow nail (10) exhibits at least one transverse groove (13; 13a) open from the introduction end of the nail, into which the shank part serving as reception part of the transverse bolt (2a) fits.

4. Device according to claim 2 or 3, characterized in that the marrow nail exhibits continuous transverse holes (4) for further transverse bolts (2b, 2c), whilst the axes of said transverse holes lie in an axial plane of the marrow nail, whilst the plane of the longitudinal slit (3; 43) and/or the axis of the transverse groove (13: 13a) of the tip part of the marrow nail (1; 10) is oriented in or parallel or at right angles to said axial plane.

5. Device according to any of claims 1 to 4, characterized in that the reception part of the shank of the transverse bolt (2a) is made thicker or thinner in cross-section compared to the remaining transverse bolt shank parts.

6. Device according to any of claims 1 to 4, characterized in that the transverse bolt (2a) exhibiting the reception part is an initial locking bolt provided with a terminal screwthread.

7. Device according to any of claims 1 to 4, characterized in that the transverse bolt exhibiting the reception part is a target bolt (52a) replaceable by an initial locking bolt (2a).

8. Device according to claim 1, characterized in that the shank of the transverse bolt (32a; 42a) exhibits as reception part a continuous transverse aperture (23b; 33b) into which the tip part (23a; 33a) of the marrow nail (10b), flattened or conically tapered compared to the shank cross-section of the marrow nail and serving as the introduction part, fits.

9. Device according to any of claims 1 to 8, characterized in that the reception part of the shank of the transverse bolt exhibits a circular or polyhedral cross-section.

## Revendications

1. Dispositif pour fixer des fractures d'os tubulaires, avec une broche médullaire et des chevilles de verrouillage, caractérisé par le fait qu'une première cheville transversale, servant au verrouillage primaire, est munie d'une partie réceptrice avec laquelle la région pointue de la broche médullaire, pourvue d'une partie d'introduction, peut être mise en prise par coulissement longitudinal de ladite broche perpendiculairement à la cheville transversale fixe.

2. Dispositif selon la revendication 1, caractérisé par le fait que la région pointue de la broche médullaire (1; 10) présente une fente longitudinale (3) ouverte à partir de l'extrémité d'introduction de la broche, ou bien une gorge transversale (73), fente longitudinale ou gorge transversale dans laquelle s'adapte respectivement la région du fût de la cheville transversale (2a), servant de partie réceptrice.

3. Dispositif selon la revendication 1, caractérisé par le fait que la région pointue de la broche médullaire (10) présente au moins une rainure transversale (13; 13a) ouverte à partir de l'extrémité d'introduction de la broche, et dans laquelle s'adapte la région du fût de la cheville transversale (2a), servant de partie réceptrice.

4. Dispositif selon la revendication 2 ou 3, caractérisé par le fait que la broche médullaire présente des trous transversaux ininterrompus (4) pour d'autres chevilles transversales (2b, 2c), les axes de ces trous transversaux se trouvant dans un plan axial de la broche médullaire, le plan de la fente longitudinale (3; 43) ou, respectivement, l'axe de la rainure transversale (13; 13a) de la région pointue de la broche médullaire (1; 10) s'étendant dans ce plan axial, ou bien parallèlement ou perpendiculairement à celui-ci.

5. Dispositif selon l'une des revendications 1 à 4, caractérisé par le fait que la partie réceptrice du fût de la cheville transversale (2a) est de section épaissie ou amincie par rapport aux régions de fût des autres chevilles transversales.

6. Dispositif selon l'une des revendications 1 à 4, caractérisé par le fait que la cheville transversale (2a) présentant la partie réceptrice est une cheville de verrouillage primaire, pourvue d'un filetage extrême.

7. Dispositif selon l'une des revendications 1 à 4, caractérisé par le fait que la cheville transversale présentant la partie réceptrice est une cheville de pointage (52a), pouvant être remplacée par une cheville de verrouillage primaire (2a).

8. Dispositif selon la revendication 1, caractérisé par le fait que le fût de la cheville transversale (32a; 42a) servant au verrouillage primaire présente, en tant que partie réceptrice, un orifice transversal ininterrompu (23b; 33b) dans lequel s'adapte la région pointue (23a; 33a) de la broche médullaire (10b), qui sert de partie d'introduction et est aplatie ou rétrécie coniquement par rapport à la section du fût de la broche médullaire.

9. Dispositif selon l'une des revendications 1 à 8, caractérisé par le fait que la partie réceptrice du fût de la cheville transversale présente une section ronde ou polygonale.

Fig 1

Fig. 2

Fig. 3

2a

Fig. 4

3 1

2a

0 170 787

3

Fig. 5

Fig. 6

Fig. 7

3  10  4

Fig. 8

3  4  10

Fig. 9

13  10  4

Fig. 11

3a  10a  4

Fig. 10

13  13a  10

Fig. 12

10a  3a

0 170 787

Fig. 13

Fig. 15

Fig. 14

Fig. 16

0170787

Fig. 17

43

4

10

Fig. 18

53

53

10

2a

Fig. 19

63

10c

2a

Fig. 22

Fig. 23

Fig. 24

Fig. 25

Fig. 26

Fig. 27

Fig. 28

Fig. 29

0 170 787

Fig. 21

73

4    10    4

Fig. 20

4

10

10

54

55

55